# EUROPEAN PATENT APPLICATION

(11) **EP 4 342 506 A1**
(43) Date of publication of application: **27.03.2024**
(21) Application number: 22197737.4
(22) Date of filing: 26.09.2022
(51) Int. Cl.: A61M 5/142, A61M 5/168, A61M 5/172

(54) **MEASUREMENT OF TISSUE PROPERTIES USING PRESSURE PATTERN**

(71) Applicant: Becton, Dickinson and Company, Franklin Lakes, NJ 07417-1880 (US)
(72) Inventor: BEGUIN, Steve, Rathdrum A67KW31 (IE); GIL, Ludovic, 38000 Grenoble (FR); O'REILLY, Simon, Dublin D12 VR68 (IE)
(74) Representative: Germain Maureau

(57) **Abstract**

A method of measuring tissue properties using a fluid line in fluid communication with a subcutaneous tissue includes applying a pressure profile or flow profile to the subcutaneous tissue via the fluid line, with the fluid line isolated from atmospheric pressure, measuring a pressure value or flow rate value over time in response to the applied pressure profile or applied flow profile, and calculating, using at least one processor, at least one of the following: time constant of pressure decay; fluidic resistance of tissue; permeability of tissue; mechanical compliance of tissue; osmotic pressure of interstitial fluid of tissue; capillary pressure in subcutaneous tissue; and ionic strength of interstitial fluid.

## Description

### BACKGROUND OF THE INVENTION

### Field of the Invention

The present disclosure relates to a method of measuring tissue properties using a pressure pattern.

### Description of Related Art

Wearable medical devices, such as automatic injectors, have the benefit of providing therapy to the patient at a location remote from a clinical facility and/or while being worn discretely under the patient's clothing. The wearable medical device can be applied to the patient's skin and configured to automatically deliver a dose of a pharmaceutical composition within a predetermined time period after applying the wearable medical device to the patient's skin, such as after a 27 hour delay. After the device delivers the pharmaceutical composition to the patient, the patient may subsequently remove and dispose of the device.

In certain circumstances, due to the medium the liquid is being injected, the flow of fluid leaving the device may be impaired, which can lead to increased pressure in the fluid line of the device. When the pressure rises above a certain threshold, the integrity of the fluid path may be compromised causing a leak within the device and a failure to deliver the full dose of medicament. A fluid leak within the device may also cause damage to the device and subsequent system failures as well as potential contamination concerns due to contact between the fluid and the device.

Human subcutaneous tissue is composed of various cell types, extracellular matrix (ECM) constituents, microstructures, and macroscopic arrangement of cells and ECM. Those elements contribute to the mechanical properties of the tissue. The tissue may also include the lymphatic system, blood vessels, and has intrinsic fluid absorption and retention properties. These characteristics vary among individuals, location within the body, and over time may cause variable degrees of resistance to the infusion of fluids at the site of injection. When the fluidic resistance of the tissue is too high or the absorption rate is too low for a given delivery flow rate from the device, the pressure may build up and reach valves above the threshold where the fluid line and other components may be compromised.

### SUMMARY OF THE INVENTION

In one aspect or embodiment, a method of measuring tissue properties using a fluid line in fluid communication with a subcutaneous tissue includes: applying a pressure profile or flow profile to the subcutaneous tissue via the fluid line, with the fluid line isolated from atmospheric pressure; measuring a pressure value or flow rate value over time in response to the applied pressure profile or applied flow profile; and calculating, using at least one processor, at least one of the following: time constant of negative pressure decay back to tissue rest pressure; time constant of positive pressure decay back to tissue rest pressure; fluidic resistance of tissue; permeability of tissue; mechanical compliance of tissue; osmotic pressure of interstitial fluid of tissue; capillary pressure in subcutaneous tissue; and ionic strength of interstitial fluid.

The tissue rest pressure may be about atmospheric pressure. The tissue rest pressure may be positive relative to atmospheric pressure with a pressure difference of 0-500 Pa. The tissue rest pressure may be negative relative to atmospheric pressure with a pressure difference of 0-500 Pa.

A negative pressure profile may be applied to the subcutaneous tissue, with the negative pressure profile lower than atmospheric pressure. A positive pressure profile may be applied to the subcutaneous tissue, with the positive pressure profile higher than atmospheric pressure. The negative pressure profile may be applied to the subcutaneous tissue by using an insertion mechanism to insert a needle and catheter into the subcutaneous tissue and subsequently retracting the needle from the subcutaneous tissue while leaving the catheter in fluid communication with the subcutaneous tissue, with the catheter is in fluid communication with the fluid line. The pressure value or the flow rate value may be measured using a pressure sensor measuring a pressure within the fluid line. The pressure profile or flow profile may be applied by pumping a fluid through the fluid line. The method may further include delivering a fluid to the subcutaneous tissue at a predetermined flow rate based on the calculated permeability of the tissue.

In a further aspect or embodiment, a drug delivery device includes a power source, a reservoir configured to receive a fluid, a fluid line in fluid communication with the reservoir, a pump configured to deliver the fluid from the reservoir to the fluid line, and a microcontroller including at least one processor programmed or configured to cause the device to: apply a pressure profile or flow profile to the subcutaneous tissue via the fluid line, the fluid line isolated from atmospheric pressure; measure a pressure value or flow rate value over time in response to the applied pressure profile or applied flow profile; and calculate at least one of time constant of negative pressure decay back to tissue rest pressure; time constant of positive pressure decay back to tissue rest pressure, fluidic resistance of tissue, permeability of tissue, mechanical compliance of tissue, osmotic pressure of interstitial fluid of tissue, capillary pressure in subcutaneous tissue, and ionic strength of interstitial fluid.

The device may further include an insertion mechanism including a catheter and a needle, where the microcontroller is configured to cause a negative pressure profile to be applied to the subcutaneous tissue by using the insertion mechanism to insert the needle and the catheter into the subcutaneous tissue and subsequently retracting the needle from the subcutaneous tissue while leaving the catheter in fluid communication with the subcutaneous tissue. The catheter is in fluid communication with the fluid line. The device may further include a pressure sensor, where the pressure value or the flow rate value is measured using the pressure sensor measuring a pressure within the fluid line. The microcontroller may be configured to apply the pressure profile or the flow profile by pumping a fluid through the fluid line using the pump.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings.
**FIG. 1** is a perspective view of a drug delivery device according to one aspect or embodiment of the present application.
**FIG. 2** is a perspective view of the drug delivery device of FIG. 1, with a top cover removed.
**FIG. 3** is a schematic view of the drug delivery device of FIG. 1.
**FIG. 4** is a flowchart of a method of measuring tissue properties according to one aspect or embodiment of the present application.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION OF THE INVENTION

Spatial or directional terms, such as "left", "right", "inner", "outer", "above", "below", and the like, are not to be considered as limiting as the invention can assume various alternative orientations.

All numbers used in the specification and claims are to be understood as being modified in all instances by the term "about". By "about" is meant a range of plus or minus ten percent of the stated value. As used in the specification and the claims, the singular form of "a", "an", and "the" include plural referents unless the context clearly dictates otherwise. The terms "first", "second", and the like are not intended to refer to any particular order or chronology, but instead refer to different conditions, properties, or elements. By "at least" is meant "greater than or equal to".

As used herein, "at least one of' is synonymous with "one or more of'. For example, the phrase "at least one of A, B, and C" means any one of A, B, or C, or any combination of any two or more of A, B, or C. For example, "at least one of A, B, and C" includes one or more of A alone; or one or more of B alone; or one or more of C alone; or one or more of A and one or more of B; or one or more of A and one or more of C; or one or more of B and one or more of C; or one or more of all of A, B, and C.

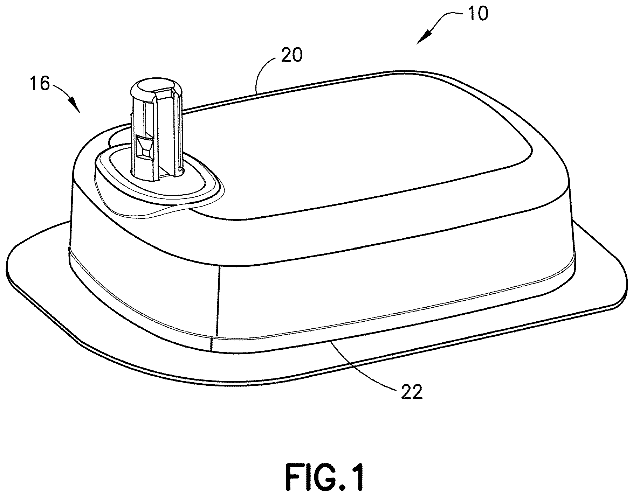

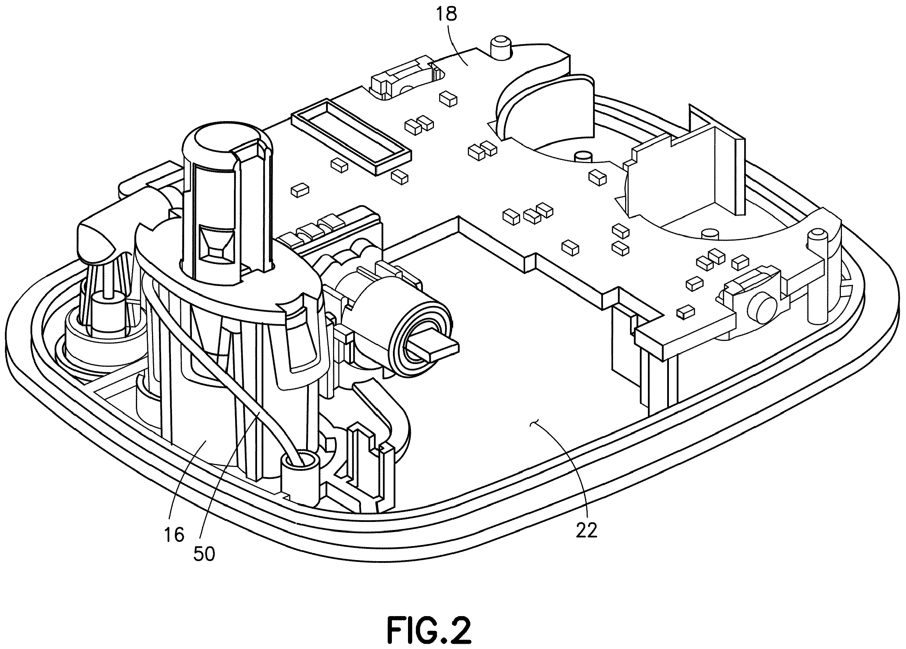

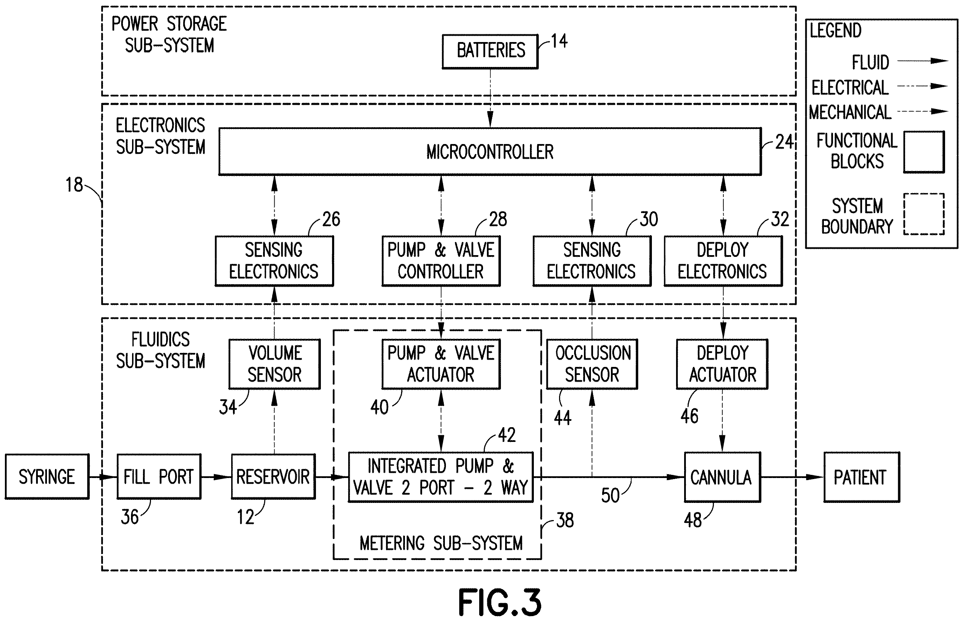

Referring to FIGS. 1-3, a drug delivery device 10 includes a reservoir 12, a power source 14, an insertion mechanism 16, control electronics 18, a cover 20, and a base 22. In one aspect or embodiment, the drug delivery device 10 is a wearable automatic injector, such as an insulin or bone marrow stimulant delivery device. The drug delivery device 10 may be mounted onto the skin of a patient and triggered to inject a pharmaceutical composition from the reservoir 12 into the patient. The drug delivery device 10 may be pre-filled with the pharmaceutical composition, or it may be filled with the pharmaceutical composition by the patient or medical professional prior to use.

The drug delivery device 10 is configured to deliver a dose of a pharmaceutical composition, e.g., any desired medicament, into the patient's body by a subcutaneous injection at a slow, controlled injection rate. Exemplary time durations for the delivery achieved by the drug delivery device 10 may range from about 5 minutes to about 60 minutes, but are not limited to this exemplary range. Exemplary volumes of the pharmaceutical composition delivered by the drug delivery device 10 may range from about 0.1 milliliters to about 10 milliliters, but are not limited to this exemplary range. The volume of the pharmaceutical composition delivered to the patient may be adjusted.

Referring again to FIGS. 1-3, in one aspect or embodiment, the power source 14 is a DC power source including one or more batteries. The control electronics 18 include a microcontroller 24, sensing electronics 26, a pump and valve controller 28, sensing electronics 30, and deployments electronics 32, which control the actuation of the drug delivery device 10. The drug delivery device 10 includes a fluidics sub-system that includes the reservoir 12, volume sensor 34 for the reservoir 12, a reservoir fill port 36, and a metering system 38 including a pump and valve actuator 40 and a pump and valve mechanism 42. The fluidic sub-system may further include an occlusion sensor 44, a deploy actuator 46, a cannula 48 for insertion into a patient's skin, and a fluid line 50 in fluid communication with the reservoir 12 and the cannula 48. The cannula 48 may include a needle and/or catheter. In one aspect or embodiment, the insertion mechanism 16 is configured to move the cannula 48 from a retracted position positioned entirely within the device 10 to an extended position where the cannula 48 extends outside of the device 10. The drug delivery device 10 may operate in the same manner as discussed in U.S. Patent No. 10,449,292 to Pizzochero et al.

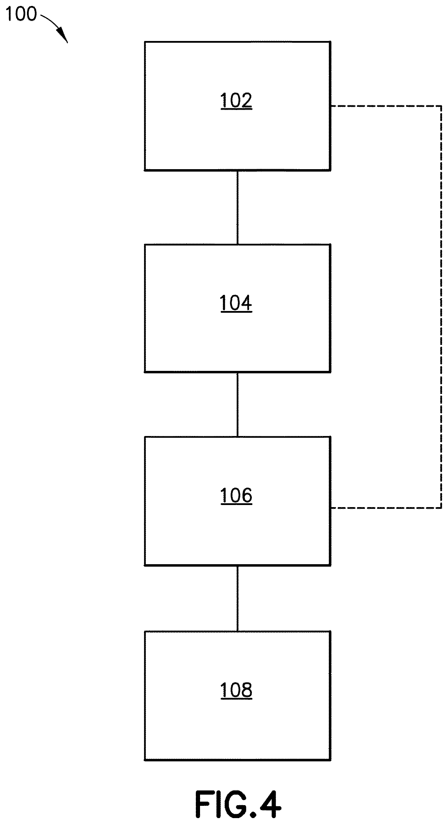

Referring to FIG. 4, in one aspect or embodiment, a method 100 of measuring tissue properties using a fluid line 50 in fluid communication with a subcutaneous tissue includes: applying a pressure profile or flow profile 102 to the subcutaneous tissue via the fluid line 50, with the fluid line 50 isolated from atmospheric pressure; measuring a pressure value or flow rate value over time 104 in response to the applied pressure profile or applied flow profile; and calculating 106, using at least one processor, at least one of the following: time constant of negative pressure decay back to atmospheric pressure; time constant of positive pressure decay back to atmospheric pressure; fluidic resistance of tissue; permeability of tissue; mechanical compliance of tissue; osmotic pressure of interstitial fluid of tissue; capillary pressure in subcutaneous tissue; and ionic strength of interstitial fluid. In one aspect or embodiment, one or more of the above properties are calculated using at least one of the following: amplitude of pressure peak (negative or positive); initial slope of the decay via linear interpolation of any combination of two or more consecutive pressure measurements; curve fitting of the equation of exponential decay of 1^{st} order Y = A^{∗}(x^-t/tau) + B, where A can be positive or negative; and curve fitting of the equation of exponential decay of higher or lower order Y = A^{∗}(x^-t/tau)^Z + B, where Z can be between 0 and infinity.

In one aspect or embodiment, the method 100 is carried out using the drug delivery device 10 discussed above, although the method 100 may be carried out using other suitable devices and arrangements. For example, the method 100 may be carried out using an infusion pump system or device rather than a wearable drug delivery device.

In one aspect or embodiment, a negative pressure profile is applied to the subcutaneous tissue, where the negative pressure profile is lower than atmospheric pressure. The negative pressure profile may be applied to the subcutaneous tissue by using the insertion mechanism 16 to insert a needle and catheter (cannula 48) into the subcutaneous tissue and subsequently retracting the needle from the subcutaneous tissue while leaving the catheter in fluid communication with the subcutaneous tissue. The catheter is in fluid communication with the fluid line 50.

In one aspect or embodiment, a positive pressure profile is applied to the subcutaneous tissue, where the positive pressure profile is higher than atmospheric pressure. In one aspect or embodiment, the tissue rest pressure is about atmospheric pressure. In one aspect or embodiment, the tissue rest pressure is positive relative to atmospheric pressure with a pressure difference of 0-500 Pa. In one aspect or embodiment, the tissue rest pressure is negative relative to atmospheric pressure with a pressure difference of 0-500 Pa.

The pressure value or the flow rate value is measured using a pressure sensor measuring a pressure within the fluid line 50. The pressure sensor may be the occlusion sensor 44 or form a portion of the occlusion sensor 44. In some aspects or embodiments, the pressure profile or flow profile is applied by pumping a fluid through the fluid line 50. The method may further include delivering a fluid to the subcutaneous tissue at a predetermined flow rate based on the calculated permeability of the tissue. In other words, the injection or infusion profile may be tuned based on the measured tissue properties to optimize the dynamics of drug absorption.

In one aspect or embodiment, the microcontroller 24 of the drug delivery device 10 includes at least one processor programmed or configured to cause the device to: apply a pressure profile or flow profile to the subcutaneous tissue via the fluid line 50; measure a pressure value or flow rate value over time in response to the applied pressure profile or applied flow profile; and calculate at least one of the following: time constant of negative pressure decay back to atmospheric pressure; time constant of positive pressure decay back to atmospheric pressure; fluidic resistance of tissue; permeability of tissue; mechanical compliance of tissue; osmotic pressure of interstitial fluid of tissue; capillary pressure in subcutaneous tissue; and ionic strength of interstitial fluid.

In one aspect or embodiment, the microcontroller 24 is configured to cause a negative pressure profile to be applied to the subcutaneous tissue by using the insertion mechanism 16 to insert the needle and the catheter (cannula 48) into the subcutaneous tissue and subsequently retracting the needle from the subcutaneous tissue while leaving the catheter in fluid communication with the subcutaneous tissue. The microcontroller 24 is configured to apply the pressure profile or the flow profile by pumping a fluid through the fluid line 50 using a pump, such as the metering system 38.

Accordingly, the method 100 and/or the drug delivery device 10 can measure tissue properties by analyzing a pressure pattern, such as decay subsequent to a positive or negative step in pressure applied in the fluid line 50. The subcutaneous tissue is a medium including multiple cell types arranged in various structure, along with extracellular matric and interstitial fluid. The different cell structures may include glomeruli, nerves, blood capillaries and vessels, lymphatic capillaries and vessels, and conjunctive tissue. The permeability of a target site of the subcutaneous tissue may vary greatly dependent on the location and patient. In some cases, the tissue may have very low permeability impacting the delivery of fluid to the tissue as well as the dynamics of absorption of the drug being administered. In other cases, the tissue may display abnormally high permeability that may impact the drug absorption leading to an absorption faster than desired for best therapeutic treatment. Injection in a tissue with low permeability may require adjusting the flowrate to avoid high pressures that could lead to leakage within the device 10. Tissue with very low permeability may lead to too slow release of the drug while tissue with very high permeability may lead to a too fast release of the drug. The ability to measure the tissue permeability before, at the early stages of, and/or during injection allows the identification of injection sites that may impact the absorption and subsequent pharmacokinetics of the drug being delivered. In particular, the ability to detect a problematic injection site at the time of the placement of the device, or soon thereafter, provides an advantage in improving the safety of the patient by avoiding delayed notification, e.g., site with low permeability that may cause an occlusion alarm to be triggered during the course of delivery. The method 100 and the drug delivery device 10 address these issues by providing for a way to measure the tissue properties and, if needed, adjust the delivery profile of the drug to the patient.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the spirit and scope of the appended claims. For example, it is to be understood that the present invention contemplates that, to the extent possible, one or more features of any embodiment can be combined with one or more features of any other embodiment.

## Claims

1. A method of measuring tissue properties using a fluid line in fluid communication with a subcutaneous tissue, the method comprising:
applying a pressure profile or flow profile to the subcutaneous tissue via the fluid line, the fluid line isolated from atmospheric pressure;
measuring a pressure value or flow rate value over time in response to the applied pressure profile or applied flow profile; and
calculating, using at least one processor, at least one of the following: time constant of negative pressure decay back to tissue rest pressure; time constant of positive pressure decay back to tissue rest pressure; fluidic resistance of tissue; permeability of tissue; mechanical compliance of tissue; osmotic pressure of interstitial fluid of tissue; capillary pressure in subcutaneous tissue; and ionic strength of interstitial fluid.

2. The method of claim 1, wherein the tissue rest pressure is about atmospheric pressure.

3. The method of claim 1, wherein the tissue rest pressure is positive relative to atmospheric pressure with a pressure difference of 0-500 Pa.

4. The method of claim 1, wherein the tissue rest pressure is negative relative to atmospheric pressure with a pressure difference of 0-500 Pa.

5. The method of claim 1, wherein a negative pressure profile is applied to the subcutaneous tissue, the negative pressure profile is lower than atmospheric pressure.

6. The method of claim 1, wherein a positive pressure profile is applied to the subcutaneous tissue, the positive pressure profile is higher than atmospheric pressure.

7. The method of claim 5, wherein the negative pressure profile is applied to the subcutaneous tissue by using an insertion mechanism to insert a needle and catheter into the subcutaneous tissue and subsequently retracting the needle from the subcutaneous tissue while leaving the catheter in fluid communication with the subcutaneous tissue, and wherein the catheter is in fluid communication with the fluid line.

8. The method of any of claims 1-7, wherein the pressure value or the flow rate value is measured using a pressure sensor measuring a pressure within the fluid line.

9. The method of any of claims 1-8, wherein the pressure profile or flow profile is applied by pumping a fluid through the fluid line.

10. The method of any of claims 1-9, further comprising:
delivering a fluid to the subcutaneous tissue at a predetermined flow rate based on the calculated permeability of the tissue.

11. A drug delivery device comprising:
a power source;
a reservoir configured to receive a fluid;
a fluid line in fluid communication with the reservoir;
a pump configured to deliver the fluid from the reservoir to the fluid line; and
a microcontroller comprising at least one processor programmed or configured to cause the device to:
apply a pressure profile or flow profile to the subcutaneous tissue via the fluid line, the fluid line isolated from atmospheric pressure;
measure a pressure value or flow rate value over time in response to the applied pressure profile or applied flow profile; and
calculate at least one of the following: time constant of negative pressure decay back to tissue rest pressure; time constant of positive pressure decay back to tissue rest pressure; fluidic resistance of tissue; permeability of tissue; mechanical compliance of tissue; osmotic pressure of interstitial fluid of tissue; capillary pressure in subcutaneous tissue; and ionic strength of interstitial fluid.

12. The device of claim 11, further comprising an insertion mechanism comprising a catheter and a needle, wherein the microcontroller is configured to cause a negative pressure profile to be applied to the subcutaneous tissue by using the insertion mechanism to insert the needle and the catheter into the subcutaneous tissue and subsequently retracting the needle from the subcutaneous tissue while leaving the catheter in fluid communication with the subcutaneous tissue, and wherein the catheter is in fluid communication with the fluid line.

13. The device of claim 11, further comprising a pressure sensor, wherein the pressure value or the flow rate value is measured using the pressure sensor measuring a pressure within the fluid line.

14. The device of claim 11, wherein the microcontroller is configured to apply the pressure profile or the flow profile by pumping a fluid through the fluid line using the pump.
